(19) Europäisches Patentamt
European Patent Office
Office européen des brevets

(11) EP 3 533 435 B1

# (12) EUROPEAN PATENT SPECIFICATION

(45) Date of publication and mention of the grant of the patent:
**13.07.2022 Bulletin 2022/28**

(21) Application number: **19156857.5**

(22) Date of filing: **13.02.2019**

(51) International Patent Classification (IPC):
***A61K 8/34*** *(2006.01)* ***A61K 8/42*** *(2006.01)*
***A61Q 5/02*** *(2006.01)* ***A61Q 5/06*** *(2006.01)*
***A61Q 5/12*** *(2006.01)* ***A61K 8/41*** *(2006.01)*
***A61K 8/44*** *(2006.01)* ***A61K 8/46*** *(2006.01)*

(52) Cooperative Patent Classification (CPC):
**A61K 8/342; A61K 8/416; A61K 8/42; A61K 8/442; A61K 8/466; A61Q 5/02; A61Q 5/065; A61Q 5/12;**
A61K 2800/596

## (54) CLEANSING AND CONDITIONING COMPOSITION FOR KERATIN FIBERS, METHOD, USE, AND KIT-OF-PARTS THEREOF

REINIGUNGS- UND KONDITIONIERUNGSZUSAMMENSETZUNG FÜR KERATINFASERN, VERFAHREN, VERWENDUNG UND TEILEKIT DAFÜR

COMPOSITION DE NETTOYAGE ET DE CONDITIONNEMENT POUR FIBRES DE KÉRATINE, PROCÉDÉ, UTILISATION ET KIT DE PIÈCES ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.02.2018 EP 18159189**

(43) Date of publication of application:
**04.09.2019 Bulletin 2019/36**

(73) Proprietor: **KAO CORPORATION**
**Tokyo 103-8210 (JP)**

(72) Inventors:
- **Picker, Andreas**
  **64297 Darmstadt (DE)**
- **Younas, Huma**
  **64297 Darmstadt (DE)**

(74) Representative: **Hoffmann Eitle**
**Patent- und Rechtsanwälte PartmbB**
**Arabellastraße 30**
**81925 München (DE)**

(56) References cited:
**EP-B1- 0 550 656**
**EP-B1- 0 784 465**
**WO-A1-97/12020**
**DE-A1- 4 309 568**
**DE-U1-202013 011 413**
**US-A1- 2014 017 185**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 533 435 B1

## Description

### Field of the invention

The present invention is directed to the field of cleansing and conditioning of keratin fibers. A composition, method for cleansing and conditioning, use of a composition, and kit-of-parts is disclosed.

### Background of the invention

Early cosmetic products used to be very simple, often comprising only few, mostly natural ingredients. They showed more or less elevated performances and were developed to fulfil one particular need. Multifunctional products were rare or resulted in compromises regarding their main purposes. Consistency or use experience did not play a role, but performance was the main target of such products.

Nowadays, consumers demand products with features beyond pure functionality. Therefore, product performance is expected to reach beyond the pure technical need such as cleansing. Consumers desire to experience a special effect during application. Relaxation, spoiling and spa feelings at home play a more and more important role when consumers choose products according to their personal taste.

There always has been a disparity between cleansing products which foam during cleansing and conditioning products which improve the cosmetic properties of keratin fibers, but often lack surprising foam. 2 in 1 cleansing conditioning products are well established on the market, but they are often impaired in their conditioning properties while providing a less creamy feel and less conditioning performance than pure conditioning products.

In WO2013/178701 conditioning compositions for hair are disclosed which comprise fatty alcohol, cationic surfactant, and N-alkylpolyhydroxyalkylamide type surfactants as non-ionic surfactant. However, the document is silent on cleansing conditioning compositions comprising a second non-ionic surfactant which results in improved cleansing properties without diminishing the conditioning properties.

Pure conditioning products (Mintel #4886209) comprising cationic surfactant, alkyl polyglycoside, and fatty alcohol are known, but they lack N-alkylpolyhydroxyalkylamide type surfactants and are not used for cleansing hair. Cleansing conditioners with similar ingredients are known (Mintel #4113911), but they still lack N-alkylpolyhydroxyalkylamide type surfactants and have less conditioning properties.

WO 97/12020A1 to a liquid laundry detergent composition comprising a surfactant system, which comprises anionic surfactants selected from the group of alkyl alkoxy sulfates and alkyl sulfates, and a quaternary ammonium surfactant having a specific formula (I).

EP 0 550 656 relates to a shampoo composition comprising a silicone-containing hair conditioning agent at a level of from 0,05%-10% by weight; said composition optionally comprising one or more adjunct surfactants, optional thickening agents and fluid carrier, said composition being characterized in that it comprises at least 1%, preferably at least 3%, by weight of a polyhydroxy fatty acid amide surfactant.

DE 4309568 A1 concerns detergent compositions comprising poly hydroxy fatty acid amides of a specific formula (I), cationic surfactans and an optional oil component.

US 2014/017185A1 relates to a conditioner in the form of an optically nontransparent dispersion containing at least one cationic surfactant, a micro emulsion containing bl) at least one alkyl glycoside and/or an alkyl oligoglycoside, b2) at least one cosurfactant which does not fall under the definition of bl), b3) an organic oil phase, and b4) water, at least one fatty alcohol, optionally further surfactants and optionally further cosmetic additives, wherein the sum of all the surfactants present in the conditioner makes up a proportion of at most 10 wt % of the conditioner.

DE 20 2013 011413 U1 relates to condintioning shampoos comprising anionic surfactants, glucamides and fatty alcohols.

EP 0 784 465 concerns cosmetic compositions comprising (a) fatty acid-N-alkyl glucamides and (b) silicone compounds, with the proviso that, when the compositions have a surfactant content of 5 to 40% by weight and alkyl sulfates and/or alkyl ether sulfates make up 80 to 99% by weight of that surfactant content, the percentage fatty acid-N-alkyl glucamide content is less than 1 or more than 20% by weight.

### Summary of the invention

The inventors of the present invention have unexpectedly found that the combination of fatty alcohols, at least two different non-ionic surfactants, and surfactants having at least one positively charged group below pH 7 yield a composition which provides cleansing and conditioning benefits to keratin fibers while delivering excellent foam in terms of creaminess and stability. Despite consumer's experience, the resulting foams are characterized by a large laminal area and a high laminar thickness factor (ratio of area of foam bubbles to total micrograph area) which is visible under the microscope wherein the inventive compositions deliver a laminar thickness factor of at least 5. In contrast to pure

cleansing compositions, the compositions according to the present invention act as deposition aid for hair direct dyes onto keratin fibers. Thus, the inventive composition possesses self-evident multifunctional properties such as cleansing and conditioning but also fulfils the desire of consumers to feel, see and experience the next level of foam creaminess. This experience is directly connected to positive emotions and thus highly appreciated in new personal care and especially hair care formulations.

**Description of the Figure**

Figure 1: Illustration of the evaluation method of the micrographs obtained in the Examples. $A_o$ is the product of x and y, whereas $A_i$ is the total area sum of all bubbles present in the micrograph.

**Detailed description of the invention**

The first object of the present invention is an aqueous cosmetic cleansing and/or conditioning composition according to Claim 1.

The second object of the invention is a method for cleansing and/or conditioning of keratin fibers, preferably human keratin fibers, more preferably human hair, characterized in that a composition as defined above is applied onto keratin fibers, massaged for 10 s to 300 s, and rinsed-off with water.

The third object of the invention is a use of a composition as defined above as deposition aid of hair direct dyes onto keratin fibers, preferably human keratin fibers, more preferably human hair.

A further object of the present invention is a kit-of-parts comprising the composition as defined above.

It is a preferred embodiment of the present invention that the composition has a pH in the range of 2 to 6, preferably in the range of 3 to 5, more preferably in the range of 3.5 to 4.5.

Suitable fatty alcohols having a linear or branched, saturated or unsaturated carbon chain length of $C_{12}$-$C_{22}$ according to the present invention are, for example, lauryl alcohol, tridecyl alcohol, myristyl alcohol, pentadecyl alcohol, cetyl alcohol, palmitoleyl alcohol, heptadecyl alcohol, stearyl alcohol, oleyl alcohol, nonadecyl alcohol, arachidyl alcohol, behenyl alcohol, and/or their mixtures, in particular cetearyl alcohol.

The composition comprises fatty alcohols at a total concentration in the range of usually 0.5% to 10% by weight, preferably 1% to 8% by weight, more preferably 2% to 6% by weight, calculated to the total of the composition.

Fatty alcohols within the meaning of the present invention are not considered as surfactants.

Suitable surfactants having one or more positively charged group(s) at a pH below 7 are selected from cationic and/or cationizable and/or zwitterionic/amphoteric surfactants, and/or their salt(s).

Suitable cationic surfactants are of quaternary ammonium structure according to the following general structure

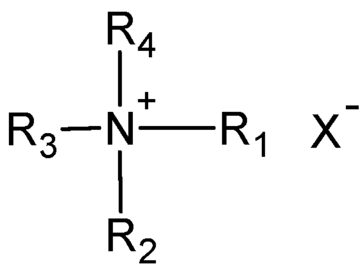

where $R_1$ is a saturated or unsaturated, branched or linear alkyl chain with $C_8$-$C_{22}$ or

$R_5$ CO NH $(CH_2)_n$

where $R_5$ is saturated or unsaturated, branched or linear alkyl chain with $C_7$-$C_{21}$ atoms and n has typical value of 1-4 or

$R_6$ CO O $(CH_2)_n$

where $R_6$ is saturated or unsaturated, branched or linear alkyl chain with $C_7$-$C_{21}$ atoms and n has typical value of 1-4, and $R_2$ is unsaturated or saturated, branched or linear alkyl chain with $C_1$-$C_{22}$ atoms or

$R_5$ CO NH $(CH_2)_n$

or

$R_6$ CO O $(CH_2)_n$

where $R_5$, $R_6$ and n are same as above.

$R_3$ and $R_4$ have an alkyl chain with $C_1$ to $C_4$, and $X^-$ is typically chloride, bromide, or methosulfate.

Typical examples of those ingredients are cetyl trimethyl ammonium chloride, stearyl trimonium chloride, dipalmitoyl dimonium chloride, distearyl dimethyl ammonium chloride, stearamidopropyl trimethyl ammonium chloride, dioleoylethyl dimethyl ammonium methosulfate, dioleoylethyl hydroxyethylmonium methosulfate, behenyl trimethyl ammonium chloride, and/or their mixtures.

Suitable cationizable surfactants are surfactants which carry one or more chemical group(s) that is/are at least at some point non-ionic at pH above 7, but which is/are positively charged at a pH under 7. Such groups are, for example, primary, secondary, and tertiary amino groups. It is well known to the skilled reader that the aforementioned groups are becoming ammonium groups at a pH below 7.

Suitable cationizable surfactants are, for example, according to the following general structure

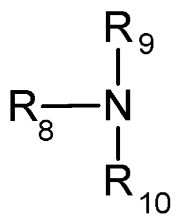

where $R_8$ is a saturated or unsaturated, straight or branched alkyl chain, optionally modified with ethoxylate and/or propxylate groups, with $C_{12}$ to $C_{22}$, $R_9$ is selected from H or straight or branched alkyl with $C_1$ to $C_4$, and $R_{10}$ is selected from H or straight or branched alkyl with $C_1$ to $C_4$.

Suitable compounds according to this structure are, for example, dodecylamine, tridecylamine, tetradecylamine, pentadecylamine, stearylamine, octylamine, oleylamine, behenylamine, stearyl methylamine, stearyl dimethylamine, octyl methylamine, octyl dimethylamine, behenyl methylamine, behenyl dimethylamine, stearyl ethylamine, stearyl ethylamine, octyl ethylamine, octyl ethylamine, behenyl ethylamine, behenyl ethylamine, stearyl propylamine, stearyl dipropylamine, octyl propylamine, octyl dipropylamine, behenyl propylamine, behenyl dipropylamine and/or their salts and/or their mixtures. The aforementioned compounds may be modified with ethoxylate and/or propoxylate groups.

Further suitable cationizable surfactants are known as alkyl amido alkyl amine surfactants and/or their salt(s) and are according to the following general structure

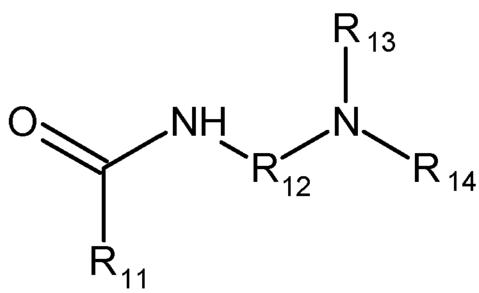

where $R_{11}$ is a saturated or unsaturated, straight or branched alkyl chain with $C_{11}$ to $C_{21}$, $R_{12}$ is a straight or branched alkyl chain with $C_1$ to $C_6$, $R_{13}$ and $R_{14}$ may be the same of different selected from H and straight or branched alkyl chain with $C_1$ to $C_4$.

Suitable compounds according to this definition are, for example, cocamidopropyl dimethylamine, stearamidopropyl dimethylamine, behenamidopropyl dimethylamine, and/or their salt(s).

Further suitable surfactants having one or more positively charged group(s) at a pH below 7 are amphoteric/zwitterionic surfactants. Amphoteric/zwitterionic surfactants may be selected from compounds according to the general structure(s)

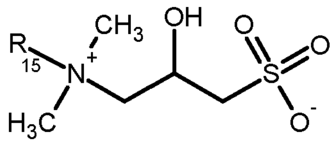

and/or

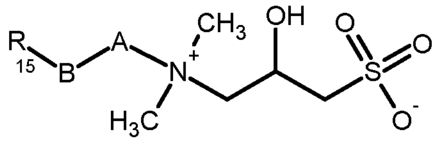

wherein $R_{15}$ is a straight or branched, saturated or unsaturated, substituted or unsubstituted alkyl chain with a carbon number of $C_{10}$ to $C_{22}$, preferably $R_{15}$ is a straight alkyl chain with a carbon number of $C_{10}$ to $C_{16}$, A is a straight alkyl chain with a carbon number of $C_1$ to $C_6$ or a branched alkyl chain with a carbon number of $C_3$ to $C_6$, preferably A is a linear alkyl chain with a carbon number of $C_3$, and B is an amide or an ester group.

Suitable compounds are known as hydroxysultaine surfactants, such as cocoamidopropyl hydroxysultaine, laurylamidopropyl hydroxysultaine, erucamidopropyl hydroxysultaine, lauryl hydroxysultaine, and cocoyl hydroxysultaine, and/or their salt(s).

Further suitable amphoteric/zwitterionic surfactants are of betaine type. Suitable compounds may be selected from alkyl betaines and/or alkylamido betaines. A preferred compound selected from alkyl betaines is lauryl betaine. A preferred compound selected from alkylamido betaines is cocamidopropyl betaine. The disclosure also relates to the salts of the compounds.

It is to be noted that the skilled person can combine the surfactants from the classes of above to create suitable surfactant mixtures.

The preferred surfactant(s) having one or more positively charged group(s) at a pH below 7 of the composition according to the present invention is/are selected from quaternary ammonium surfactant(s) and its/their salt(s), alkyl amido alkyl amine surfactant(s) and its/their salt(s), amphoteric/zwitterionic surfactants and/or its/their salt(s), and/or their mixtures.

The total concentration of surfactants having one or more positively charged group(s) at a pH below 7 is usually in the range of 0.5% to 3% by weight, preferably 0.75% to 2% by weight, and more preferably 1% to 1.5% by weight, calculated to the total of the composition.

The composition of the present invention further comprises a first non-ionic surfactant selected from N-alkylpolyhydroxyalkylamide type surfactants according to the following general formula:

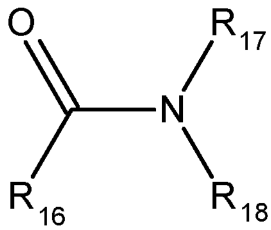

wherein $R_{16}$ is a linear or branched, saturated or unsaturated alkyl chain with $C_{11}$ to $C_{21}$, $R_{17}$ is linear or branched alkyl, or linear or branched hydroxyalkyl with $C_1$ to $C_4$, and $R_{18}$ is a linear or branched polyhydroxyalkyl chain with $C_3$ to $C_{12}$ and 3 to 10 hydroxyl groups.

Such compounds are disclosed in cosmetic compositions in WO96/27366 and their synthesis is disclosed in US1985424, US2016962, US2703798, and WO92/06984.

The preferred N-alkylpolyhydroxyalkylamide type surfactants have the following structure:

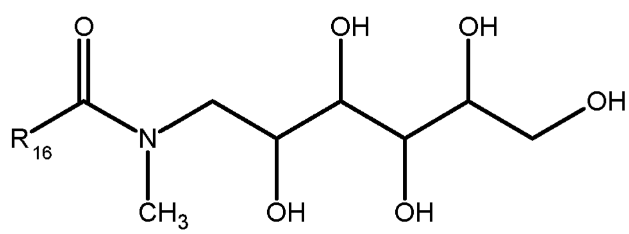

where $R_{16}$ has the same denotation as above for the general structure of N-alkylpolyhydroxyalkylamide type surfactants. The preferred surfactants as displayed above are known as N-methyl-N-acylglucamides.

The most preferred N-alkylpolyhydroxyalkylamide type surfactants are selected from lauroyl/myristoyl methyl glucamide and coco methyl glucamide.

As a second non-ionic surfactant different form the first non-ionic surfactant, the composition of the present invention comprises an alkyl glycoside or alkyl polyglycoside according to the general structure:

$$R_{23}O(R_{24}O)_tZ_x$$

Wherein Z denotes a carbohydrate with $C_5$ to $C_6$, $R_{23}$ is an alkyl group with $C_8$ to $C_{18}$, $R_{24}$ is methyl, ethyl or propyl, t ranges from 0 to 10, and x ranges from 1 to 5. Suitable compounds according to this structure are $C_9$-$C_{11}$ alkylpolyglycoside, the structures disclosed in EP-A 70 074, and JP 2015-123019A.

The preferred compounds according to the structure of above are decyl glucoside, lauryl glucoside, and coco glucoside, and the most preferred one is decyl glucoside.

Further examples for non-ionic surfactants are fatty alcohol ethoxylates of the following general structure

$$R_{25}\ (OCH_2CH_2)_{n4}\ OH$$

wherein $R_{25}$ is straight or branched, saturated or unsaturated alkyl chain which may be synthetic or natural with a C chain length in the range of 8 to 40, preferably 9 to 30 and more preferably 9 to 24 and n4 is a number in the range of 5 to 40, preferably 9 to 30.

Non-limiting suitable examples of the fatty alcohol ethoxylates are C9-11 Pareth-6, C9-11 Pareth-8, C9-15 Pareth-8, C11-13 Pareth-9, C11-13 Pareth-10, C11-15 Pareth-5, C11-15 Pareth-7, C11-15 Pareth-9, C11-15 Pareth-12, C11-15 Pareth-15, C11-15 Pareth-20, C11-15 Pareth-30, C11-15 Pareth-40, C11-21 Pareth-10, C12-13 Pareth-5, C12-13 Pareth-6, C12-13 Pareth-7, C12-13 Pareth-9, C12-13 Pareth-10, C12-13 Pareth-15, C12-13 Pareth-23, C12-14 Pareth-5, C12-14 Pareth-7, C12-14 Pareth-9, C12-14 Pareth-11, C12-14 Pareth-12, C12-15 Pareth-5, C12-15 Pareth-7, C12-15 Pareth-9, C12-15 Pareth-10, C12-15 Pareth-11, C12-15 Pareth-12, C12-16 Pareth-5, C12-16 Pareth-7, C12-16 Pareth-9, C13-15 Pareth-21, C14-15 Pareth-7, C14-15 Pareth-8, C14-15 Pareth-11, C14-15 Pareth-12, C14-15 Pareth-13, C20-22 Pareth-30, C20-40 Pareth-10, C20-40 Pareth-24, C20-40 Pareth-40, C20-40 Pareth-95, C22-24 Pareth-33, Beheneth-5, Beheneth-10, Beheneth-15, Beheneth-20, Beheneth-25, Beheneth-30, Ceteareth-5, Ceteareth-6, Ceteareth-7, Ceteareth-10, Ceteareth-11, Ceteareth-12, Ceteareth-15, Ceteareth-20, Ceteareth-25, Ceteareth-30, Ceteareth-35, Ceteareth-40, Laureth-5, Laureth-10, Laureth-15, Laureth-20, Laureth-25, Laureth-30, Laureth-40, Myreth-5, Myreth-10, Ceteth-5, Ceteth-10, Ceteth-15, Ceteth-20, Ceteth-25, Ceteth-30, Ceteth-40, Oleth-5, Oleth-10, Oleth-15, Oleth-20, Oleth-25, Oleth-30, Oleth-40, Steareth-5, Steareth-10, Steareth-15, Steareth-20, Steareth-25, Steareth-30, Steareth-35, and Steareth-40. They may also be comprised in the compositions as a mixture of more than one surfactant.

Further suitable nonionic surfactants are polypropylene glycol ethers of fatty alcohol according to general structure

$$R_{25}\ (OCH_2\text{-}CH_2\text{-}CH_2)_{n5}\ OH$$

wherein $R_{25}$ is straight or branched, saturated or unsaturated fatty alcohol which may be synthetic or natural with a C chain length in the range of 8 to 40, preferably 9 to 30 and more preferably 9 to 24 and n5 is a number in the range of 1 to 40, preferably 3 to 30.

Suitable non-limiting examples are PPG-3 Caprylyl ether, PPG-5 Caprylyl ether, PPG-10 Caprylyl ether, PPG-10 Cetyl ether, PPG-20 Cetyl ether, PPG-28 Cetyl ether, PPG-30 Cetyl ether, PPG-7 Lauryl ether, PPG-10 Lauryl ether, PPG-10 Oleyl ether, PPG-20 Oleyl ether, PPG-23 Oleyl ether, PPG-30 Oleyl ether, PPG-11 Stearyl ether and PPG-15 Stearyl ether.

Further suitable nonionic surfactants are polyethylene glycol fatty acid esters of the following general structure

$$R_{26}\ C(O)\ (OCH_2CH_2)_{n6}\ OH$$

wherein $R_{26}$ is straight or branched, saturated or unsaturated alkyl group which may be synthetic or natural with a C chain length in the range of 7 to 39, preferably 9 to 29 and more preferably 9 to 23 and n6 is a number in the range of 5 to 40, preferably 9 to 30.

Suitable non-limiting examples are PEG-8 Behenate, PEG-8 Caprate, PEG-8 Caprylate, PEG-5 Cocoate, PEG-8 Cocoate, PEG-9 Cocoate, PEG-10 Cocoate, PEG-15 Cocoate, PEG-6 Isopalmitate, PEG-6 Isostearate, PEG-8 Isostearate, PEG-9 Isostearate, PEG-10 Isostearate, PEG-12 Isostearate, PEG-20 Isostearate, PEG-30 Isostearate, PEG-40 Isostearate, PEG-6 Laurate, PEG-8 Laurate, PEG-9 Laurate, PEG-10 Laurate, PEG-12 Laurate, PEG-14 Laurate, PEG-20 Laurate, PEG-30 Laurate, PEG-8 Myristate, PEG-20 Myristate, PEG-5 Oleate, PEG-6 Oleate, PEG-7 Oleate, PEG-8 Oleate, PEG-9 Oleate, PEG-10 Oleate, PEG-11 Oleate, PEG-12 Oleate, PEG-15 Oleate, PEG-20 Oleate, PEG-30 Oleate, PEG-32 Oleate, PEG-6 Palmitate, PEG-18 Palmitate, PEG-20 Palmitate, PEG-5 Stearate, PEG-6 Stearate, PEG-7 Stearate, PEG-8 Stearate, PEG-9 Stearate, PEG-10 Stearate, PEG-12 Stearate, PEG-14 Stearate, PEG-15

Stearate, PEG-20 Stearate, PEG-25 Stearate, PEG-30 Stearate, PEG-35 Stearate and PEG-40 Stearate.

Further suitable nonionic surfactants are polypropylene glycol fatty acid esters of the following general structure

$$R_{27}\ C(O)\ (OCH_2\text{-}CH_2\text{-}CH_2)_{n8}\ OH$$

wherein $R_{27}$ is straight or branched, saturated or unsaturated alkyl group which may be synthetic or natural with a C chain length in the range of 7 to 39, preferably 9 to 29 and more preferably 9 to 23 and n8 is a number in the range of 1 to 40, preferably 9 to 30.

Suitable non-limiting examples are PPG-15 Isostearate, PPG-9 Laurate, PPG-26 Oleate and PPG-36 Oleate.

Further suitable nonionic surfactants are polyethylene glycol and polypropylene glycol ether of fatty alcohols of the following general structure

$$R_{28}\ (OCH_2\text{-}CH_2\text{-}CH_2)_{n9}\ (OCH_2CH_2)_{n10}\ OH$$

wherein $R_{28}$ is straight or branched, saturated or unsaturated alkyl group which may be synthetic or natural with a C chain length in the range of 7 to 39, preferably 9 to 29 and more preferably 9 to 23 and n9 and n10 may be the same or different and are a number in the range of 1 to 40.

Further suitable nonionic surfactants are ethoxylated vegetable oils. Well-known and commonly used examples are ethoxylated castor oil such as PEG-40 hydrogenated castor oil or and PEG-60 hydrogenated castor oil.

The composition of the present invention comprises a second non-ionic surfactant selected from alkyl glycosides or alkyl polyglycosides of the the above-shown formula, more preferably they are selected from decyl glucoside, lauryl glucoside, and coco glucoside, and further more preferably it is decyl glucoside.

The total concentration of non-ionic surfactants is in the range of 0.1% to 7.5% by weight, preferably 0.5% to 5% by weight, more preferably 0.75% to 3% by weight, calculated to the total of the composition.

The composition of the present invention comprises the first non-ionic surfactant to second non-ionic surfactant at a weight ratio in the range of usually 10 to 0.1, preferably 8 to 0.2, and more preferably 6 to 0.25.

The composition may further comprise linear and/or cyclic non-aminated silicones and/or non-aminated siliconols.

Suitable non-aminated silicones and/or non-aminated siliconols are dimethicone, dimethiconol, polydimethylsiloxane (DC fluid ranges from Dow Corning), arylated silicones such as phenyl methicone, phenyl trimethicone, diphenyl dimethicone, diphenylsiloxy phenyl trimethicone, tetramethyl tetraphenyl trisiloxane, triphenyl trimethicone, and trimethyl pentaphenyl trisiloxane, aqueous emulsions of divinyldimethicone/dimethicone copolymer, preferably with a viscosity of higher than $1 \times 10^8$ $mm^2/s$, more preferably higher than $1.1 \times 10^8$ $mm^2/s$ measured at 0.01 Hz and at 20°C with a Brookfield viscometer and an appropriate spindle.

The composition may further comprise aminosilicone(s), preferably selected from a compound according to the general structure

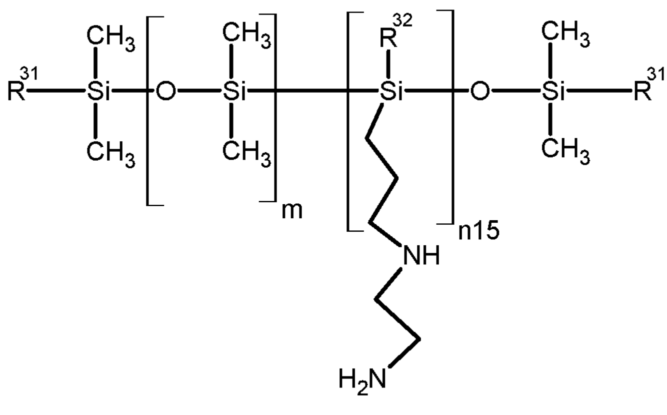

Wherein $R^{31}$ is selected from OH, $OCH_3$, and/or O-Si-$(CH_3)_3$, $R^{32}$ is selected from $CH_3$, $OCH_3$, O-(Si-$(CH_3)_2$)x-$R^{33}$, and/or O-Si-$(CH_3)_3$, with the provision that if $R^{31}$ or $R^{32}$ are selected from O-Si-$(CH_3)_3$, then all other $R^{32}$ or $R^{33}$ are selected from O-Si-$(CH_3)_3$ and/or $OCH_3$, and x is an integer from 1 to 200. m and n15 are integer numbers independently of each other and in the range of 1 to 200. Special reference is made to the aminosilicones sold by Wacker Corporation under the trade name Belsil ADM 6102E and Belsil ADM 8020VP, the ones sold by by Shin-Etsu Corp. under the trade name X-52-2265, and the ones sold by Dow Corning Corp. under the trade name DC 969.

The composition of the present invention comprises one or more silicone compound(s) at a total concentration

in the range of usually 0.01% to 1% by weight, preferably it is 0.1% to 0.5% by weight, calculated to the total of the composition.

The composition of the present invention may comprise a cationic conditioning polymer.

Suitable cationic polymers are those of best known with their INCI category name Polyquaternium.

Typical examples of those are Polyquaternium 1, Polyquaternium 2, Polyquaternium 4, Polyquaternium 5, Polyquaternium 6, Polyquaternium 7, Polyquaternium 8, Polyquaternium 9, Polyquaternium 10, Polyquaternium 11, Polyquaternium 12, Polyquaternium 13, Polyquaternium 14, Polyquaternium 15, Polyquaternium 16, Polyquaternium 17, Polyquaternium 18, Polyquaternium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquaternium 49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquaternium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, Polyquaternium 86, and Polyquaternium 87.

It has further been found out that especially those of cationic cellulose type polymers known as Polymer JR type from Amerchol such as Polyquaternium 10 or cationic galactomannans such as cationic guar gum known with trade name Jaguar from Rhone-Poulenc which are chemically for example guar hydroxypropyl trimonium chloride and cationic tara gum and its derivatives known with INCI name Caesalpinia spinosa hydroxypropyltrimonium chloride, are preferred ones. Furthermore, chitosan and chitin can also be included in the compositions as cationic natural polymers. In this context reference is also made to the cationic polymers disclosed in DE 25 21 960, DE 28 11 010, 30 44 738 and DE 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, cationic Caesalpinia spinosa gum derivatives, polyquaternium 6, polyquaternium 7, polyquaternium 10, polyquaternium 37, polyquaternium 67 and polyquaternium 70.

The cationic polymers also include the quaternized products of graft polymers from organopolysiloxanes and polyethyl oxazolines described in EP-A 524 612 and EP-A 640643.

Compositions may comprise cationic polymer at a concentration of usually 0.01 to 5%, preferably 0.02 to 4%, more preferably 0.05 to 3% and most preferably 0.1 to 2.5% by weight, calculated to the total of the composition.

In cases where a cationic silicone compound is added to the composition, the narrower concentration limitation for silicone compounds applies to the cationic silicone compounds as well.

The composition of the present invention may further comprise anionic cleansing surfactants. The total concentration of anionic cleansing surfactants in the composition of the present invention is in the range of usually 0.01% by weight to 2% by weight, preferably in the range of 0.1% to 1% by weight, more preferably in the range of 0.15% to 0.75% by weight.

Suitable anionic cleansing surfactants are alkyl sulfate or preferably ethoxylated alkyl ether sulfate surfactant or mixtures thereof with an alkyl chain length of $C_{10}$ to $C_{22}$.

Suitable surfactants are laureth sulfates, coceth sulfate, pareth sulfate, capryleth sulphate, myreth sulfate, oleth sulfate, deceth sulfate, trideceth sulfate, coco sulphate, $C_{10}$-$C_{16}$ alkyl sulphate, $C_{11}$-$C_{15}$ alkyl sulphate, $C_{12}$-$C_{18}$ alkyl sulphate, $C_{12}$-$C_{15}$ alkyl sulphate, $C_{12}$-$C_{16}$ alkyl sulphate, $C_{12}$-$C_{13}$ alkyl sulfate, lauryl sulphate, myrystyl sulphate, palm kernel sulphate, cetearyl sulfate, cetyl sulphate, decyl sulphate, oleyl sulphate, behenyl sulphate and/or their salts. All of the aforementioned anionic surfactants may or may not be ethoxylated at various degrees.

Cations for the surfactants may be selected from sodium, potassium, magnesium and/or ammonium.

The most suitable anionic cleansing surfactant is sodium laureth sulfate with 1-5 ethylene oxide units.

In a preferred embodiment of the composition of the present invention, the composition is free of anionic cleansing surfactants.

The composition of the present invention further may comprise one or more thickening agent(s), preferably selected from thickening polymers, more preferably from associative and/or non-associative thickening polymers.

In a preferred embodiment of the present invention, the composition comprises one or more thickening polymers selected from anionic, nonionic, cationic and amphoteric polymers, preferably selected from polymers resulting in a solution and/or dispersion with a viscosity of at least 500 mPa•s measured at a polymer concentration of 1% by weight in water and at 20°C with a Brookfield viscometer and spindle 4, such as at 10 rpm for 1 min.

Suitable polymers are cellulose polymers, alginates, polysaccharides and acrylic acid polymers, preferably methyl cellulose, ethyl cellulose, hydroxyethylcellulose, methylhydroxyethylcellulose, methylhydroxypropylcellulose, carboxymethyl cellulose, alginic acids, sodium alginates, ammonium alginates, calcium alginates, gum arabic, guar gum or xanthan gum, dehydroxanthan gum or acrylic acid polymers known with the CTFA adopted name Carbomer and its derivatives.

The preferred polymers are dehydroxanthan gum, xanthan gum, and polymeric anionic thickeners, namely Carbomer and its derivatives.

Of particular advantageous use are thickeners which are commonly known as associative thickeners. Preferred are copolymers and/or crosspolymers which comprise an acrylate and/or methacrylate monomer unit and at least one more hydrophobic unit such as alkyl chains. Examples are acrylates/c10-30 alkyl acrylate crosspolymer, acrylates/steareth-20 methacrylate copolymer, acrylates/stearyl acrylate/dimethicone methacrylate copolymer, acrylates/beheneth-25 methacrylate copolymer, acrylates/lauryl acrylate/stearyl acrylate/ ethylamine oxide methacrylate copolymer.

The composition preferably comprises thickening agents at a total concentration in the range of usually 0.1% to 5%, preferably, 0.2% to 3%, more preferably 0.25% to 2.5% and most preferably 0.3% to 2% by weight calculated to the total of the composition.

The viscosity of the composition of the present invention may be adjusted to a viscosity usually in the range of 10,000 mPas to 50,000 mPas, preferably in the range of 12,000 mPas to 40,000 mPas, more preferably in the range of 15,000 mPas to 30,000 mPas, measured with a Brookfield viscometer at 10 rpm with spindle number 4 at 20°C.

The composition may further comprise hair direct dyes selected from cationic and/or non-ionic hair direct dyes.

Suitable cationic dyes are in principle those available on the market for cosmetic hair colouring applications. For this purpose, special reference is made to the PCT application WO 95/15144 of Ciba-Geigy AG. Some examples to those are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic red 51, Basic Yellow 87, HC Blue 17 and Basic Orange 31. The most preferred ones are Basic red 51, Basic Yellow 87 and Basic Orange 31 sold by BASF, HC Blue 17, Basic Blue 124.

Suitable neutral dyes including nitro dyes are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2-Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

The composition may comprise one or more hair direct dye(s) at a total concentration of usually 0.001% to 10% by weight, preferably 0.005% to 7.5% by weight, and more preferably 0.01% to 5% by weight, calculated to the total of the composition. The composition can also comprise a mixture of several direct dyes, i.e. a cationic and/or nonionic hair direct dyes. In such a case the dyes may be mixed at any ratio with each other.

The following examples are to illustrate the invention, but not to limit it.

## EXAMPLES

### Example 1

The following compositions were prepared by conventional formulation techniques:

| Ingredient | Inventive comp. 1 [% by weight] | Inventive comp. 2 [% by weight] | Comparative comp. 1 [% by weight] | Comparative comp. 2 [% by weight] |
|---|---|---|---|---|
| **Cetearyl alcohol** | 6.0 | 6.0 | 6.0 | 6.0 |
| **Cetrimonium chloride** | 1.5 | 1.5 | 1.5 | 1.5 |
| **Decyl glucoside** | 1.5 | - | 3.0 | - |
| **Lauryl glucoside** | - | 2.0 | - | - |
| **Lauroyl/myristoyl methyl glucamide** | 1.5 | - | - | 3.0 |

(continued)

| Ingredient | Inventive comp. 1 [% by weight] | Inventive comp. 2 [% by weight] | Comparative comp. 1 [% by weight] | Comparative comp. 2 [% by weight] |
|---|---|---|---|---|
| **Coco methyl glucamide** | - | 1.0 | - | - |
| **Water** | Ad 100.0 | | | |

The pH of the compositions was adjusted to 4.5 with lactic acid.

Viscosities of the compositions were measured with a Brookfield viscometer with spindle 4 at 20°C. Inventive compositions 1 and 2 had viscosities in the range of 10,000 mPas to 20,000 mPas, whereas the comparative compositions had viscosities in the range of 5,000 mPas to less than 10,000 mPas.

The compositions from above were then diluted with water in a ratio of 1:10 for foam analysis. 100 mL of the diluted compositions were foamed for 30 s with a paddle stirrer (500 rpm) in a 800 mL glass beaker. The resulting foam was collected on a microscope glass slide and allowed to settle for 5 min. The foam was then observed under a microscope at 10 x magnification and micrographs were recorded. Thus, the resulting micrographs are planar projections of a 3D foam. The micrographs were then further analyzed with Imagej software wherein the diameter d of the foam bubbles in the micrograph was determined. Each foam bubble was then assigned a circular area $A_i$ by using the equation $A_i = \frac{\pi}{4} d^2$. This is exemplarily shown in Fig. 1 where each foam bubble receives an area and a serial index i. Furthermore, the rectangular area of the micrograph was measured and assigned a value $A_o$, wherein $A_o$ is the product of x and y as displayed in Fig. 1 and refers to the total area of the micrograph. For the present experiments, the evaluated micrograph area $A_o$ was 8 mm$^2$ and kept exactly the same between the treatment groups. Then a Lamellar Thickness Factor (LTF) was calculated by the following equation: $LTF = \frac{A_o}{\sum_{n=i}^{i} A_i}$, wherein the calculated areas for all measured bubbles were added to constitute $A_i$. It is to be noted that a high LTF denoted stable, rich, and creamy foam whereas a low LTF denoted a less stable, rich, and creamy foam. The following results by using this method were obtained:

| Paramter | Inventive comp. 1 | Inventive comp. 2 | Comparative comp. 1 | Comparative comp. 2 |
|---|---|---|---|---|
| **LTF** | 8.3 | 5.4 | 1.3 | 1.8 |

As a result, the LTF was found to be above 5 for the inventive compositions and below 2 for the comparative compositions. A high LTF corresponds to a large laminar area between small foam bubbles. Thus, the inventive compositions had such large areas which are associated with high foam stability and high creaminess.

**EXAMPLE 2**

The following composition according to the invention was prepared:

| | % **by weight** |
|---|---|
| Behenyl alcohol | 10.0 |
| Behentrimonium chloride | 1.5 |
| Coco glucoside | 2.25 |
| Lauroyl/myristoyl methyl glucamide | 0.75 |
| Stearylamidopropyl-dimethylamine | 0.5 |
| Hydroxyethylcellulose | 0.25 |
| Basic Red 51 | 0.15 |
| Polyquaternium 10 | 0.50 |
| Sodium chloride | 1.0 |
| Water | ad 100.0 |

The pH of the composition is adjusted to 3.5 with malic acid.

2 g of the composition was applied onto bleached human keratin fibers, massaged for 60 s and then rinsed-off with water. The hair streak was then blow dried. As a result, the hair streak treated with the inventive composition was dyed intensively red, as judged by the naked human eye.

The following examples are within the scope of the present invention.

**EXAMPLE 3**

| | % by weight |
|---|---|
| Cetearyl alcohol | 2.0 |
| Behenamidopropyl-dimethylamine | 1.0 |
| Lauryl glucoside | 1.0 |
| Stearyl methyl glucamide | 1.0 |
| Acrylates/C10-C30 alkyl acrylate crosspolymer | 0.10 |
| Basic Yellow 87 | 0.25 |
| Polyquaternium 37 | 0.50 |
| EDTA tetrasodium salt | 0.25 |
| Phenoxyethanol | 0.20 |
| 4-aminobenzoic acid | 0.15 |
| $\alpha$-tocopherol | 0.05 |
| Sodium chloride | 1.0 |
| Water | ad 100.0 |

The pH of the composition is adjusted to 5.5 with glyoxylic acid.

**EXAMPLE 4**

| | % by weight |
|---|---|
| Behenyl alcohol | 5.0 |
| Coco glucoside | 2.25 |
| Lauroyl/myristoyl methyl glucamide | 0.75 |
| Stearylamidopropyl-dimethyl amine | 0.5 |
| Hydroxyethylcellulose | 0.25 |
| Basic Red 51 | 0.15 |
| Polyquaternium 10 | 0.50 |
| Sodium chloride | 1.0 |
| Water | ad 100.0 |

The pH of the composition is adjusted to 3.5 with lactic acid.

**EXAMPLE 5**

| | % by weight |
|---|---|
| Behenyl alcohol | 8.0 |
| Behentrimonium chloride | 1.5 |
| Coco glucoside | 0.75 |
| Lauroyl/myristoyl methyl glucamide | 2.25 |
| Stearylamidopropyl-dimethyl amine | 0.5 |
| Hydroxyethylcellulose | 0.25 |

(continued)

| | % by weight |
|---|---|
| Basic Yellow 87 | 0.15 |
| Polyquaternium 10 | 0.50 |
| Sodium chloride | 1.0 |
| Water | ad 100.0 |

The pH of the composition is adjusted to 3.5 with lactic acid.

**EXAMPLE 6**

| | % by weight |
|---|---|
| Cetearyl alcohol | 5.0 |
| Cocoamidopropyl betaine | 2.5 |
| Coco glucoside | 0.75 |
| Lauroyl/myristoyl methyl glucamide | 2.0 |
| Hydroxyethylcellulose | 0.25 |
| Basic Yellow 87 | 0.15 |
| Polyquaternium 16 | 0.10 |
| Sodium chloride | 1.0 |
| Water | ad 100.0 |

The pH of the composition is adjusted to 3.5 with citric acid.

**EXAMPLE 7**

| | % by weight |
|---|---|
| Stearyl alcohol | 10.0 |
| Lauryl hydroxysultaine | 2.0 |
| Coco glucoside | 1.0 |
| Lauroyl/myristoyl methyl glucamide | 1.5 |
| Hydroxyethylcellulose | 0.25 |
| Basic Yellow 87 | 0.15 |
| Polyquaternium 6 | 0.10 |
| Sodium chloride | 1.0 |
| Water | ad 100.0 |

The pH of the composition is adjusted to 4.5 with lactic acid.

## Claims

1. An aqueous cosmetic cleansing and/or conditioning composition having a pH in the range of 1 to 7 **characterized in that** it comprises:

    - one or more fatty alcohol(s) having a linear or branched, saturated or unsaturated carbon chain length of $C_{12}$-$C_{22}$,
    - one or more surfactant(s) having one or more positively charged group(s) at a pH below 7,
    - a first non-ionic surfactant selected from N-alkylpolyhydroxyalkylamide type surfactants,

- a second non-ionic surfactant being different from the first non-ionic surfactant and being different from fatty alcohols,

wherein the total concentration of first and second non-ionic surfactants is in the range of 0.1% to 7.5% by weight, preferably 0.5% to 5% by weight, more preferably 0.75% to 3% by weight, calculated to the total of the composition,
**characterized in that** it comprises alkyl glycosides or polyglycosides of the following formula:

$$R_{23}O(R_{24}O)_tZ_x$$

wherein Z denotes a carbohydrate with $C_5$ to $C_6$, $R_{23}$ is an alkyl group with $C_8$ to $C_{18}$, $R_{24}$ is methyl, ethyl or propyl, t ranges from 0 to 10, and x ranges from 1 to 5.

2. The composition according to claim 1 **characterized in that** the total concentration of fatty alcohols is in the range of 0.5% to 10% by weight, preferably 1% to 8% by weight, more preferably 2% to 6% by weight, calculated to the total of the composition.

3. The composition according to claims 1 and/or 2 **characterized in that** one or more surfactant(s) having one or more positively charged group(s) at a pH below 7 is/are selected from quaternary ammonium surfactant(s) and its/their salt(s), alkyl amido alkyl amine surfactant(s) and its/their salt(s), amphoteric/zwitterionic surfactant(s) and/or its/their salt(s), and/or their mixtures.

4. The composition according to any of the preceding claims **characterized in that** the total concentration of surfactants having a net positive charge at a pH below 7 is in the range of 0.5% to 3% by weight, preferably 0.75% to 2% by weight, and more preferably 1% to 1.5% by weight, calculated to the total of the composition.

5. The composition according to any of the preceding claims **characterized in that** the weight ratio of first non-ionic surfactant to second non-ionic surfactant is in the range of 10 to 0.1, preferably 8 to 0.2, and more preferably 6 to 0.25.

6. The composition according to any of the preceding claims **characterized in that** the first non-ionic surfactant is selected from lauroyl/myristoyl methyl glucamide and/or coco methyl glucamide.

7. The composition according to any of the preceding claims **characterized in that** it comprises a cationic conditioning polymer and/or a silicone compound, and preferably the total concentration of silicone compounds is in the range of 0.01% to 1% by weight, preferably it is 0.1% to 0.5% by weight, calculated to the total of the composition.

8. The composition according to any of the preceding claims **characterized in that** it comprises anionic cleansing surfactants at a concentration in the range of 0.01% by weight to 2% by weight, preferably in the range of 0.1% to 1% by weight, more preferably in the range of 0.15% to 0.75% by weight.

9. The composition according to any of the preceding claims **characterized in that** it has a pH in the range of 2 to 6, preferably in the range of 3 to 5, more preferably in the range of 3.5 to 4.5.

10. The composition according to any of the preceding claims **characterized in that** it comprises a thickening agent, preferably selected from thickening polymers, more preferably from associative and/or non-associative thickening polymers.

11. The composition according to any of the preceding claims **characterized in that** it has a viscosity in the range of 10,000 mPas to 50,000 mPas, preferably in the range of 12,000 mPas to 40,000 mPas, more preferably in the range of 15,000 mPas to 30,000 mPas, measured with a Brookfield viscometer at 10 rpm with spindle number 4 at 20°C.

12. The composition according to any of the preceding claims **characterized in that** it comprises hair direct dyes selected from cationic and/or non-ionic hair direct dyes.

13. Method for cleansing and/or conditioning of keratin fibers, preferably human keratin fibers, more preferably human hair, **characterized in that** a composition as defined in the claims 1 to 12 is applied onto keratin fibers, massaged for 10 s to 300 s, and rinsed-off with water.

14. Use of a composition as defined in the claims 1 to 12 as deposition aid for hair direct dyes on keratin fibers, preferably human keratin fibers, more preferably human hair.

## Patentansprüche

1. Wässrige kosmetische Reinigungs- und/oder Konditionierungszusammensetzung mit einem pH-Wert im Bereich von 1 bis 7, **dadurch gekennzeichnet, dass** sie umfasst:

   - einen oder mehrere Fettalkohol(e) mit einer linearen oder verzweigten, gesättigten oder ungesättigten Kohlenstoffkettenlänge von $C_{12}$-$C_{22}$,
   - ein oder mehrere Tensid(e) mit einer oder mehreren positiv geladenen Gruppe(n) bei einem pH-Wert unter 7,
   - ein erstes nichtionisches Tensid, das ausgewählt ist aus Tensiden vom Typ N-Alkylpolyhydroxyalkylamid,
   - ein zweites nicht-ionisches Tensid, das sich von dem ersten nicht-ionischen Tensid unterscheidet und von Fettalkoholen verschieden ist,

   wobei die Gesamtkonzentration des ersten und des zweiten nicht-ionischen Tensids im Bereich von 0,1 bis 7,5 Gew.-%, bevorzugt 0,5 bis 5 Gew.-%, besonders bevorzugt 0,75 bis 3 Gew.-%, berechnet auf die Gesamtmenge der Zusammensetzung, liegt,
   **dadurch gekennzeichnet, dass** sie Alkylglykoside oder Polyglykoside der folgenden Formel umfasst:

   $$R_{23}O\ (R_{24}O)\ _tZ_x$$

   worin Z ein Kohlenhydrat mit $C_5$ bis $C_6$ bezeichnet, $R_{23}$ eine Alkylgruppe mit $C_8$ bis $C_{18}$ ist, $R_{24}$ Methyl, Ethyl oder Propyl ist, t im Bereich von 0 bis 10 liegt und x im Bereich von 1 bis 5 liegt.

2. Zusammensetzung gemäß Anspruch 1, **dadurch gekennzeichnet, dass** die Gesamtkonzentration der Fettalkohole im Bereich von 0,5 bis 10 Gew.-%, bevorzugt 1 bis 8 Gew.-%, besonders bevorzugt 2 bis 6 Gew.-%, berechnet auf die Gesamtmenge der Zusammensetzung, liegt.

3. Zusammensetzung gemäß Anspruch 1 und/oder 2, **dadurch gekennzeichnet, dass** ein oder mehrere Tensid(e) mit einer oder mehreren positiv geladenen Gruppe(n) bei einem pH-Wert unter 7 ausgewählt ist/sind aus quaternären Ammonium-Tensiden und deren Salzen, Alkylamidoalkylamin-Tensiden und deren Salzen, amphoteren/zwitterionischen Tensiden und/oder deren Salzen und/oder deren Mischungen.

4. Zusammensetzung gemäß mindestens einem der vorhergehenden Ansprüche, **dadurch gekennzeichnet, dass** die Gesamtkonzentration an Tensiden mit einer positiven Nettoladung bei einem pH-Wert unter 7 im Bereich von 0,5 bis 3 Gew.-%, bevorzugt 0,75 bis 2 Gew.-% und besonders bevorzugt 1 bis 1,5 Gew.-%, berechnet auf die Gesamtmenge der Zusammensetzung, liegt.

5. Zusammensetzung gemäß mindestens einem der vorhergehenden Ansprüche, **dadurch gekennzeichnet, dass** das Gewichtsverhältnis von dem ersten nicht-ionischen Tensid zu dem zweiten nicht-ionischen Tensid im Bereich von 10 bis 0,1, bevorzugt 8 bis 0,2 und besonders bevorzugt 6 bis 0,25 liegt.

6. Zusammensetzung gemäß mindestens einem der vorhergehenden Ansprüche, **dadurch gekennzeichnet, dass** das erste nicht-ionische Tensid ausgewählt ist aus Lauroyl/Myristoyl-Methylglucamid und/oder Coco-Methylglucamid.

7. Zusammensetzung gemäß mindestens einem der vorhergehenden Ansprüche, **dadurch gekennzeichnet, dass** sie ein kationisches Konditionierungspolymer und/oder eine Siliconverbindung umfasst, wobei die Gesamtkonzentration der Siliconverbindungen bevorzugt im Bereich von 0,01 bis 1 Gew.-%, bevorzugt 0,1 bis 0,5 Gew.-%, berechnet auf die Gesamtmenge der Zusammensetzung, liegt.

8. Zusammensetzung gemäß mindestens einem der vorhergehenden Ansprüche, **dadurch gekennzeichnet, dass** sie anionische Reinigungstenside in einer Konzentration im Bereich von 0,01 Gew.-% bis 2 Gew.-%, bevorzugt im Bereich von 0,1 Gew.-% bis 1 Gew.-%, besonders bevorzugt im Bereich von 0,15 Gew.-% bis 0,75 Gew.-% umfasst.

9. Zusammensetzung gemäß mindestens einem der vorhergehenden Ansprüche, **dadurch gekennzeichnet, dass**

sie einen pH-Wert im Bereich von 2 bis 6, bevorzugt im Bereich von 3 bis 5, besonders bevorzugt im Bereich von 3,5 bis 4,5 aufweist.

**10.** Zusammensetzung gemäß mindestens einem der vorhergehenden Ansprüche, **dadurch gekennzeichnet, dass** sie ein Verdickungsmittel umfasst, das bevorzugt ausgewählt ist aus Verdickungspolymeren, besonders bevorzugt aus assoziativen und/oder nicht-assoziativen Verdickungspolymeren.

**11.** Zusammensetzung gemäß mindestens einem der vorhergehenden Ansprüche, **dadurch gekennzeichnet, dass** sie eine Viskosität im Bereich von 10.000 mPas bis 50.000 mPas, bevorzugt im Bereich von 12.000 mPas bis 40.000 mPas, besonders bevorzugt im Bereich von 15.000 mPas bis 30.000 mPas, gemessen mit einem Brookfield-Viskosimeter bei 10 U/min mit Spindel Nummer 4 bei 20°C, aufweist.

**12.** Zusammensetzung gemäß mindestens einem der vorhergehenden Ansprüche, **dadurch gekennzeichnet, dass** sie Haardirektfarbstoffe umfasst, die ausgewählt sind aus kationischen und/oder nicht-ionischen Haardirektfarbstoffen.

**13.** Verfahren zum Reinigen und/oder Konditionieren von Keratinfasern, bevorzugt menschlichen Keratinfasern, besonders bevorzugt menschlichen Haaren, **dadurch gekennzeichnet, dass** eine Zusammensetzung wie in mindestens einem der Ansprüche 1 bis 12 definiert auf Keratinfasern aufgetragen, 10 s bis 300 s lang einmassiert und mit Wasser ausgespült wird.

**14.** Verwendung einer Zusammensetzung wie in mindestens einem der Ansprüche 1 bis 12 definiert als Abscheidungshilfsmittel für Haardirektfarbstoffe auf Keratinfasern, bevorzugt menschlichen Keratinfasern, besonders bevorzugt menschlichen Haaren.

**Revendications**

**1.** Composition cosmétique aqueuse de nettoyage et/ou de conditionnement présentant un pH dans la plage de 1 à 7, **caractérisée en ce qu'**elle comprend :

- un ou plusieurs alcools gras présentant une longueur de chaîne de carbone linéaire ou ramifiée, saturée ou insaturée en $C_{12}$-$C_{22}$,
- un ou plusieurs tensioactifs présentant un ou plusieurs groupes chargés positivement à un pH inférieur à 7,
- un premier tensioactif non ionique choisi parmi des tensioactifs de type N-alkylpolyhydroxyalkylamide,
- un second tensioactif non ionique différent du premier tensioactif non ionique et différent des alcools gras,

dans laquelle la concentration totale des premier et second tensioactifs non ioniques est dans la plage de 0,1 % à 7,5 % en poids, de préférence de 0,5 % à 5 % en poids, plus préférentiellement de 0,75 % à 3 % en poids, calculée sur le total de la composition, **caractérisée en ce qu'**elle comprend des alkylglycosides ou polyglycosides de la formule suivante :

$$R_{23}O\ (R_{24}O)\ _tZ_x$$

dans laquelle Z désigne un glucide en $C_5$ à $C_6$, $R_{23}$ est un groupe alkyle en $C_8$ à $C_{18}$, $R_{24}$ est un méthyle, éthyle ou propyle, t est dans la plage de 0 à 10, et x est dans la plage de 1 à 5.

**2.** Composition selon la revendication 1, **caractérisée en ce que** la concentration totale en alcools gras est dans la plage de 0,5 % à 10 % en poids, de préférence de 1% à 8 % en poids, plus préférentiellement de 2 % à 6 % en poids, calculée par rapport au total de la composition.

**3.** Composition selon les revendications 1 et/ou 2, **caractérisée en ce qu'** un ou plusieurs tensioactifs présentant un ou plusieurs groupes chargés positivement à un pH inférieur à 7 est/sont choisis parmi un/des tensioactifs d'ammonium quaternaire et un ou des sels de ceux-ci, un/des tensioactifs alkylamidoalkylamines et un ou des sels de ceux-ci, un/ou des tensioactifs amphotères/zwitterioniques et/ou un ou des sels de ceux-ci, et/ou des mélanges de ceux-ci.

**4.** Composition selon l'une quelconque des revendications précédentes, **caractérisée en ce que** la concentration

totale d'agents tensioactifs présentant une charge nette positive à un pH inférieur à 7 est dans la plage de 0,5 % à 3 % en poids, de préférence de 0,75 % à 2 % en poids, et plus préférentiellement de 1 % à 1,5 % en poids, calculée par rapport au total de la composition.

**5.** Composition selon l'une quelconque des revendications précédentes, **caractérisée en ce que** le rapport pondéral du premier tensioactif non ionique au second tensioactif non ionique est dans la plage de 10 à 0,1, de préférence de 8 à 0,2, et plus préférentiellement de 6 à 0,25.

**6.** Composition selon l'une quelconque des revendications précédentes, **caractérisée en ce que** le premier tensioactif non ionique est choisi parmi lauroyl/myristoyl méthyl glucamide et/ou coco méthyl glucamide.

**7.** Composition selon l'une quelconque des revendications précédentes, **caractérisée en ce qu'**elle comprend un polymère conditionneur cationique et/ou un composé siliconé, et de préférence la concentration totale en composés siliconés dans la plage de 0,01 % à 1 % en poids, de préférence de 0,1 % à 0,5 % en poids, calculée par rapport au total de la composition.

**8.** Composition selon l'une quelconque des revendications précédentes, **caractérisée en ce qu'**elle comprend des tensioactifs de nettoyage anioniques à une concentration dans la plage de 0,01 % en poids à 2 % en poids, de préférence dans la plage de 0,1 % à 1 % en poids, plus préférentiellement dans la plage de 0,15 % à 0,75 % en poids.

**9.** Composition selon l'une quelconque des revendications précédentes, **caractérisée en ce qu'**elle présente un pH dans la plage de 2 à 6, de préférence dans la plage de 3 à 5, plus préférentiellement dans la plage de 3,5 à 4,5.

**10.** Composition selon l'une quelconque des revendications précédentes, **caractérisée en ce qu'** elle comprend un agent épaississant, de préférence choisi parmi des polymères épaississants, plus préférentiellement parmi des polymères épaississants associatifs et/ou non associatifs.

**11.** Composition selon l'une quelconque des revendications précédentes, **caractérisée en ce qu'** elle présente une viscosité dans la plage de 10 000 mPas à 50 000 mPas, de préférence dans la plage de 12 000 mPas à 40 000 mPas, plus préférentiellement dans la plage de 15 000 mPas à 30 000 mPas, mesurée avec un viscosimètre Brookfield à 10 tr/min avec la broche numéro 4 à 20°C.

**12.** Composition selon l'une quelconque des revendications précédentes, **caractérisée en ce qu'** elle comprend des colorants directs capillaires choisis parmi des colorants directs capillaires cationiques et/ou non ioniques.

**13.** Procédé de nettoyage et/ou de conditionnement de fibres de kératine, de préférence des fibres de kératine humaine, plus préférentiellement des cheveux humains, **caractérisé en ce qu'**une composition telle que définie dans les revendications 1 à 12 est appliquée sur les fibres de kératine, massée pendant 10 s à 300 s, et rincée avec de l'eau.

**14.** Utilisation d'une composition telle que définie dans les revendications 1 à 12 comme auxiliaire de dépôt de colorants directs capillaires sur des fibres de kératine, de préférence des fibres de kératine humaines, plus préférentiellement des cheveux humains.

Figure 1

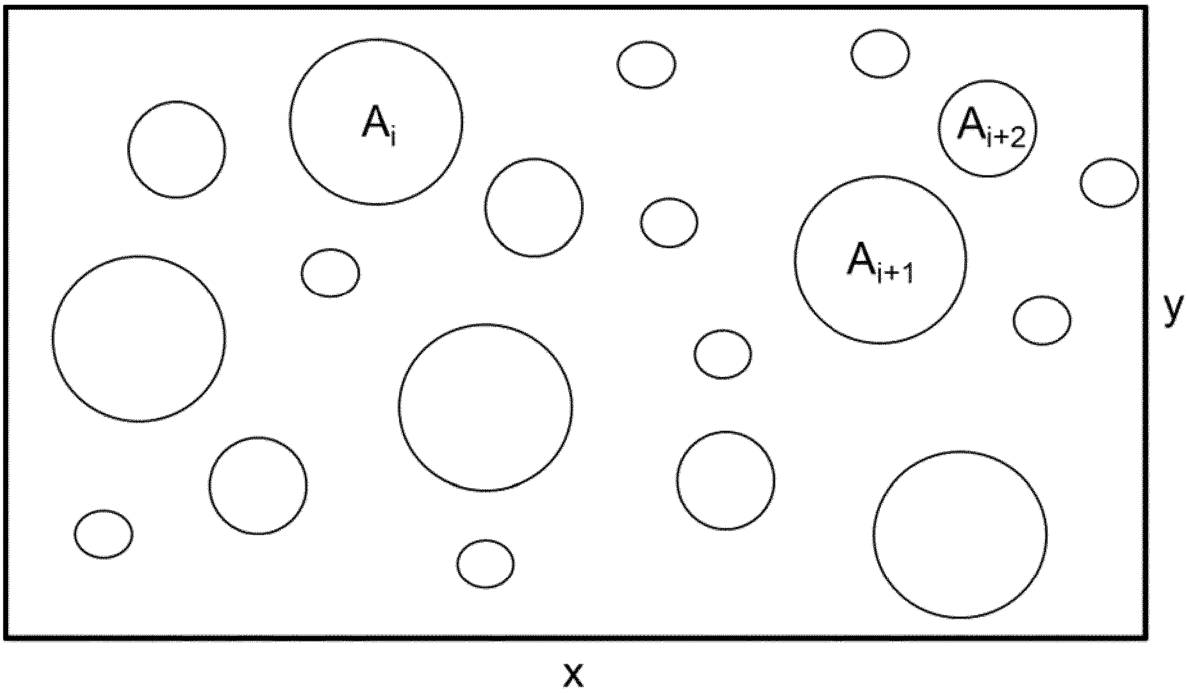

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013178701 A
- WO 9712020 A1
- EP 0550656 A
- DE 4309568 A1
- US 2014017185 A1
- DE 202013011413 U1
- EP 0784465 A
- WO 9627366 A
- US 1985424 A
- US 2016962 A
- US 2703798 A
- WO 9206984 A
- EP 70074 A
- JP 2015123019 A
- DE 2521960
- DE 28110103044738
- DE 3217059
- EP 337354 A
- EP 524612 A
- EP 640643 A
- WO 9515144 A